# EUROPEAN PATENT SPECIFICATION

(11) **EP 2 615 119 B1**
(45) Date of publication and mention of the grant of the patent: **26.10.2016**
(21) Application number: 12150870.9
(22) Date of filing: 12.01.2012
(51) Int. Cl.: C08F 6/00, A61L 15/22, A61L 15/60, C08F 8/44, C08J 3/00, C08L 33/02, F25B 15/02, A61L 15/24, C08J 3/075, C08F 220/06, F25B 15/06

(54) **Cooling neutralized acrylic acid by means of an absorption chiller**
Kühlung neutralisierter Acrylsäure mittels Absorptionskälteanlage
Refroidissement d'acide acrylique neutralisé au moyen d'un dispositif frigorifique d'absorption

(43) Date of publication of application: 17.07.2013
(73) Proprietor: Evonik Degussa GmbH, 45128 Essen (DE)
(72) Inventor: Vorholt, Herbert, 45721 Haltern am See (DE); Rudolph, Henry, 47809 Krefeld (DE); van Stiphoudt, Manfred, 47906 Kempen (DE); Reimann, Armin, 47877 Willich (DE); Jung, Detlef, 47918 Toenisvorst (DE); Beerhorst, Martin, 48356 Nordwalde (DE); Grupe, Heinz-Peter, 47803 Krefeld (DE); Becker, Volker, 46147 Oberhausen (DE)

(56) References cited:
- WO-A1-02/22717
- DE-A1- 19 909 653
- DE-A1-102005 042 604
- US-A1- 2010 010 184
- US-A1- 2011 126 563

## Description

The invention relates to a process for the preparation of water-absorbent polymer particles.

Superabsorbers are water-insoluble, crosslinked polymers which are able to absorb large amounts of aqueous fluids, especially body fluids, more especially urine or blood, with swelling and the formation of hydrogels, and to retain such fluids under a certain pressure. By virtue of those characteristic properties, such polymers are chiefly used for incorporation into sanitary articles, such as, for example, baby's nappies/diapers, incontinence products or sanitary towels.

The preparation of superabsorbers is generally carried out by free-radical polymerisation of acid-group-carrying monomers in the presence of crosslinkers, it being possible for polymers having different absorber properties to be prepared by the choice of the monomer composition, the crosslinkers and the polymerisation conditions and of the processing conditions for the hydrogel obtained after the polymerisation (for details see, for example, Modern Superabsorbent Polymer Technology, FL Buchholz, GT Graham, Wiley-VCH, 1998). The hydrogel obtained after the polymerisation is usually comminuted, dried and classified in order to obtain a particulate superabsorber with a well defined particles size distribution. In a further process step these superabsorbent particles are often surface crosslinked in order to improve the absorption behavior. For this purpose the particles are mixed with an aqueous solution containing a surface crosslinking agent and optionally further additives and the thus obtained mixture is heat treated in order to promote the crosslinking reaction.

The acid-group-carrying monomers can be polymerized in the presence of the crosslinkers in a batch process or in a continuous process. Both in continuous and in batchwise polymerization, partially neutralized acrylic acid is typically used as the monomer. Suitable neutralization processes are described, for example, in EP 0 372 706 A2, EP 0 574 260 A1, WO 2003/051415 A1, EP 1 470 905 A1, WO 2007/028751 A1, WO 2007/028746 A1 and WO 2007/028747 A1.

DE 19909653, WO 02/22717, und US 2010010184 describe the production of superabsorbents by polymerization, drying, grinding, sizing and surface treating steps.

US 2011/126563 describes absorption chillers in general.

EP 0 372 706 A2 describes a three-stage neutralization process in which, in a first stage, acrylic acid and sodium hydroxide solution are metered in simultaneously, so as to attain a degree of neutralization of from 75 to 100 mol %, and the degree of neutralization is raised in a second stage to from 100.1 to 110 mol % in order to hydrolyze the diacrylic acid present as an impurity in the acrylic acid used, and a degree of neutralization of from 20 to 100 mol % is established in a third stage by addition of further acrylic acid. EP 0 574 260 A1 discloses, at page 7 lines 38 to 41, that the neutralization advantageously involves initially charging sodium hydroxide solution and then adding acrylic acid with cooling. WO 2003/051415 A1 teaches a process for preparing water-absorbing polymers, in which the monomer solution has a minimum temperature of 40°C. EP 1 470 905 A1 describes, in the examples, the continuous neutralization of acrylic acid immediately upstream of the polymerization reactor. Owing to the heat of neutralization, the temperature rises to 95°C. WO 2007/028751 A1 discloses a process for continuous neutralization, wherein the temperature peaks which occur in the course of neutralization are minimized. WO 2007/028746 A1 describes a continuously monitored neutralization process. WO 2007/028747 A1 teaches the use of a pre-neutralized monomer solution for preparation of monomer solutions with a different degree of neutralization.

A further process for neutralizing acrylic acid is disclosed in WO 2010/003884 A1. According to this process a monomer solution comprising acrylic acid is at least partially neutralized and the heat of the neutralization is at least partially removed using an indirect heat exchanger by means of a cooling medium, wherein the specific cooling performance of the heat exchanger is less than 10 W/cm².

The disadvantage of theses prior art processes for neutralizing acrylic acid and cooling the thus obtained aqueous solution of partially neutralized acrylic acid can be seen in the fact that the overall process for preparing the superabsorbers, which process not only comprises the neutralization step in which neutralization heat is generated, but also subsequent process steps such as the drying of the particulate polymer gel obtained after the radical polymerization or the surface crosslinking reaction, is still improvable with respect to the energy balance. For example in the drying and surface crosslinking steps, energy is withdrawn in the form of steam which often is not sufficiently recirculated in the overall process.

In general, the object of the invention consists in contributing to overcoming the disadvantages arising from the prior art in the context of the production of superabsorbers.

A further object consists in providing a process for the preparation of water-absorbent polymer particles, which compared to the processes known from the state of the art is characterized by a more advantageous energy balance. An "*advantageous energy, balance*" in the sense of the present invention is preferably meant to describe a process wherein heat that is generated in one or more process steps is recirculated as much as possible into other process steps in which heat is required, thus minimizing the amount of heat that has to be introduced into the process externally.

A contribution to the solution of these objects is made by a process for the preparation of water-absorbent polymer particles, comprising the process steps of
(i) preparing an aqueous monomer solution comprising at least partially neutralized, monoethylenically unsaturated monomers bearing carboxylic acid groups (α1) and at least one crosslinker (α3);
(ii) optionally adding fine particles of a water-absorbent polymer to the aqueous monomer solution;
(iii) adding a polymerization initiator or a at least one component of a polymerization initiator system that comprises two or more components to the aqueous monomer solution;
(iv) decreasing the oxygen content of the aqueous monomer solution;
(v) charging the aqueous monomer solution into a polymerization reactor;
(vi) polymerizing the monomers in the aqueous monomer solution in the polymerization reactor;
(vii) discharging the polymer gel strand out of the polymerization reactor and optionally comminuting the polymer gel thereby obtaining polymer gel particles;
(viii) drying the polymer gel particles;
(ix) grinding the dried polymer gel particles thereby obtaining particulate water-absorbent polymer particles;
(x) sizing the grinded water-absorbent polymer particles; and
(xi) treating the surface of the grinded and sized water-absorbent polymer particles;
wherein in process step (i) the preparation of the aqueous monomer solution comprises the step of:
(ia) at least partially neutralizing acrylic acid by mixing acrylic acid with an aqueous solution of sodium hydroxide, thereby obtaining an aqueous solution of the at least partially neutralized acrylic acid;
(ib) cooling the aqueous solution of the at least partially neutralized acrylic acid with a cooling liquid in a heat exchanger, wherein the cooling liquid used in the heat exchanger has been cooled in an absorption chiller in which at least a part of the heat of the heated cooling liquid obtained in the heat exchanger (2) is consumed by the evaporation of a refrigerant liquid in a vacuum that is created by the absorption of a refrigerant vapour into an absorbent.

The process according to the present invention is preferably a continuous process in which the aqueous monomer solution is continuously provided and is continuously fed into the polymerization reactor. The hydrogel obtained is continuously discharged out of the polymerization reactor and is continuously comminuted, dried, grinded and classified in the subsequent process steps. This continuous process may, however, be interrupted in order to, for example,
- substitute certain parts of the process equipment, like the belt material of the conveyor belt if a conveyor belt is used as the polymerization reactor,
- clean certain parts of the process equipment, especially for the purpose of removing polymer deposits in tanks or pipes, or
- start a new process when water-absorbent polymer particles with other absorption characteristics have to be prepared.

Water-absorbent polymer particles which are preferred according to the invention are particles that have an average particle size in accordance with WSP 220.2 (test method of "*Word Strategic Partners*" EDANA and INDA) in the range of from 10 to 3,000 µm, preferably 20 to 2,000 µm and particularly preferably 150 to 850 µm. In this context, it is particularly preferable for the content of polymer particles having a particle size in a range of from 300 to 600 µm to be at least 30 wt.-%, particularly preferably at least 40 wt.-% and most preferably at least 50 wt.-%, based on the total weight of the water-absorbent polymer particles.

In process step (i) of the process according to the present invention an aqueous monomer solution containing partially neutralized, monoethylenically unsaturated monomers bearing carboxylic acid groups (α1) and at least one crosslinker (α3) is prepared.

Preferred monoethylenically unsaturated monomers bearing carboxylic acid groups (α1) are those cited in DE 102 23 060 A1 as preferred monomers (α1), whereby acrylic acid is particularly preferred.

It is preferred according to the present invention that the water-absorbent polymer produced by the process according to the invention comprises monomers bearing carboxylic acid groups to at least 50 wt. %, preferably to at least 70 wt. % and further preferably to at least 90 wt. %, based on the dry weight. It is particularly preferred according to the invention, that the water-absorbent polymer produced by the process according to the invention is formed from at least 50 wt. %, preferably at least 70 wt. % of acrylic acid, which is preferably neutralized to at least 20 mol %, particularly preferably to at least 50 mol %. The concentration of the partially neutralized, monoethylenically unsaturated monomers bearing carboxylic acid groups (α1) in the aqueous monomer solution that is provided in process step (i) is preferably in the range between 10-60 wt.-%, preferably 20 to 50 wt.-% and most preferably between 30 to 40 wt.-%, based on the total weight of the aqueous monomer solution.

The aqueous monomer solution may also comprise monoethylenically unsaturated monomers (α2) which are copolymerizable with (α1). Preferred monomers (α2) are those monomers which are cited in DE 102 23 060 A1 as preferred monomers (α2), whereby acrylamide is particularly preferred.

Preferred crosslinkers (α3) according to the present invention are compounds which have at least two ethylenically unsaturated groups in one molecule (crosslinker class I), compounds which have at least two functional groups which can react with functional groups of the monomers (α1) or (α2) in a condensation reaction (= condensation crosslinkers), in an addition reaction or a ring-opening reaction (cross-linker class II), compounds which have at least one ethylenically unsaturated group and at least one functional group which can react with functional groups of the monomers (α1) or (α2) in a condensation reaction, an addition reaction or a ring-opening reaction (crosslinker class III), or polyvalent metal cations (cross-linker class IV). Thus with the compounds of crosslinker class I a crosslinking of the polymer is achieved by radical polymerisation of the ethylenically unsaturated groups of the crosslinker molecules with the monoethylenically unsaturated monomers (α1) or (α2), while with the compounds of crosslinker class II and the polyvalent metal cations of crosslinker class IV a crosslinking of the polymer is achieved respectively via condensation reaction of the functional groups (crosslinker class II) or via electrostatic interaction of the polyvalent metal cation (crosslinker class IV) with the functional groups of the monomer (α1) or (α2). With compounds of cross-linker class III a cross-linking of the polymers is achieved correspondingly by radical polymerisation of the ethylenically unsaturated groups as well as by condensation reaction between the functional groups of the cross-linkers and the functional groups of the monomers (α1) or (α2).

Preferred crosslinkers (α3) are all those compounds which are cited in DE 102 23 060 A1 as crosslinkers (α3) of the crosslinker classes I, II, III and IV, whereby
- as compounds of crosslinker class I, N, N'-methylene bisacrylamide, polyethyleneglycol di(meth)acrylates, triallylmethylammonium chloride, tetraallylammonium chloride and allylnonaethyleneglycol acrylate produced with 9 mol ethylene oxide per mol acrylic acid are particularly preferred, and
- and as compounds of crosslinker class IV, Al₂ (SO₄)₃ and its hydrates are particularly preferred.

Preferred water-absorbent polymers produced by the process according to the invention are polymers which are crosslinked by crosslinkers of the following crosslinker classes or by crosslinkers of the following combinations of crosslinker classes respectively: I, II, III, IV, I II, I III, I IV, I II III, I II IV, I III IV, II III IV, II IV or III IV.

Further preferred water-absorbent polymers produced by the process according to the invention are polymers which are crosslinked by any of the crosslinkers disclosed in DE 102 23 060 A1 as crosslinkers of crosslinker classes I, whereby N,N'-methylene bisacrylamide, polyethyleneglycol di(meth)acrylates, triallylmethylammonium chloride, tetraallylammonium chloride and allylnonaethyleneglycol acrylate produced from 9 mol ethylene oxide per mol acrylic acid are particularly preferred as crosslinkers of crosslinker class I.

The aqueous monomer solution may further comprise water-soluble polymers (α4). Preferred water-soluble polymers (α4) include partly or completely saponified polyvinyl alcohol, polyvinylpyrrolidone, starch or starch derivatives, polyglycols or polyacrylic acid. The molecular weight of these polymers is not critical, as long as they are water-soluble. Preferred water-soluble polymers (α4) are starch or starch derivatives or polyvinyl alcohol. The water-soluble polymers (α4), preferably synthetic, such as polyvinyl alcohol, can not only serve as a graft base for the monomers to be polymerized. It is also conceivable for these water-soluble polymers to be mixed with the hydrogel or the already dried, water-absorbent polymer.

The aqueous monomer solution can furthermore also comprise auxiliary substances (α5), these auxiliary substances including, in particular, complexing agents, such as, for example, EDTA.

The relative amount of monomers (α1) and (α2) and of crosslinking agents (α3) and water-soluble polymers (α4) and auxiliary substances (α5) in the aqueous monomer solution is preferably chosen such that the water-absorbent polymer structure obtained in process step x) after drying is based
- to the extent of 20-99.999 wt.-%, preferably to the extent of 55-98.99 wt.-% and particularly preferably to the extent of 70-98.79 wt.-% on monomers (α1),
- to the extent of 0-80 wt.-%, preferably to the extent of 0-44.99 wt.-% and particularly preferably to the extent of 0.1-44.89 wt.-% on the monomers (α2),
- to the extent of 0-5 wt.-%, preferably to the extent of 0.001-3 wt.-% and particularly preferably to the extent of 0.01-2.5 wt.-% on the crosslinking agents (α3),
- to the extent of 0-30 wt.-%, preferably to the extent of 0-5 wt.-% and particularly preferably to the extent of 0.1-5 wt.-% on the water-soluble polymers (α4),
- to the extent of 0-20 wt.-%, preferably to the extent of 0-10 wt.-% and particularly preferably to the extent of 0.1-8 wt.-% on the auxiliary substances (α5), and
- to the extent of 0.5-25 wt.-%, preferably to the extent of 1-10 wt.-% and particularly preferably to the extent of 3-7 wt.-% on water (α6)
the sum of the amounts by weight (α1) to (α6) being 100 wt.-%.

Optimum values for the concentration in particular of the monomers, crosslinking agents and water-soluble polymers in the monomer solution can be determined by simple preliminary experiments or from the prior art, in particular the publications US 4,286,082, DE 27 06 135 A1, US 4,076,663, DE 35 03 458 A1, DE 40 20 780 C1, DE 42 44 548 A1, DE 43 33 056 A1 and DE 44 18 818 A1.

In process step (ii) fine particles of a water-absorbent polymer may optionally be added to the aqueous monomer solution.

Water-absorbent fine particles are preferably water-absorbent polymer particles the composition of which corresponds to the composition of the above described water-absorbent polymer particles, wherein it is preferred that at least 90 wt.-% of the water-absorbent fine particles, preferably at least 95 wt.-% of the water-absorbent fine particles and most preferred at least 99 wt.-% of the water-absorbent polymer particles have a particle size of less than 200 µm, preferably less than 150 µm and particular preferably less than 100 µm.

In a preferred embodiment of the process according to the present invention the water-absorbent fine particles which may optionally be added to the aqueous monomer solution in process step (ii) are fine particles which are obtained in process step (x) of the process according to the present invention and which are thus recycled.

The fine particles can be added to the aqueous monomer solution by means of any mixing device the person skilled of the art would consider as appropriate for this purpose. In a preferred embodiment of the present invention, which is especially useful if the process is performed continuously as described above, the fine particles are added to the aqueous monomer solution in a mixing device in which a first stream of the fine particles and a second stream of the aqueous monomer solution are directed continuously, but from different directions, onto a rotating mixing device. Such a kind of mixing setup can be realised in a so called "*Rotor Stator Mixer*" which comprise in their mixing area a preferably cylindrically shaped, non-rotating stator, in the centre of which a likewise preferably cylindrically shaped rotor is rotating. The walls of the rotor as well as the walls of the stator are usually provided with notches, for example notches in the form of slots, through which the mixture of fine particles and aqueous monomer solution can be sucked through and thus can be subjected to high shear forces.

In this context it is particularly preferred that the first stream of the fine particles an the second stream of the aqueous monomer solution form an angle δ in the range from 60 bis 120°, more preferred in the range from 75 bis 105°, even more preferably in the range from 85 bis 95° and most preferred from an angle of about 90°. It is also preferred that the stream of the mixture of fine particles and aqueous monomer solution that leaves the mixer and the first stream of fine particles that enters the mixer form an angle ε in the range from 60 bis 120°, preferably in the range from 75 bis 105°, even more preferred in the range from 85 bis 95° and most preferred form an angle of about 90°.

Such a kind of mixing set up can, for example, be realized by means of mixing devices which are disclosed in DE-A-25 20 788 and DE-A-26 17 612. Concrete examples of mixing devices which can be used to add the fine particles to the aqueous monomer solution in process step (ii) of the present invention are the mixing devices which can be obtained by the IKA^{®} Werke GmbH & Co. KG, Staufen, Germany, under designations MHD 2000/4, MHD 2000/05, MHD 2000/10, MDH 2000/20, MHD 2000/30 und MHD 2000/50, wherein the mixing device MHD 2000/20 is particularly preferred. Further mixing devices which can be used are those offered by ystral GmbH, Ballrechten-Dottingen, Germany, for example under designation "Conti TDS", or by Kinematika AG, Luttau, Switzerland, for example under the trademark Megatron®.

The amount of fine particles that may be added to the aqueous monomer solution in process step (ii) is preferably in the range from 0.1 to 15 wt.-%, even more preferred in the range from 0.5 to 10 wt.-% Gew.-% and most preferred in the range from 3 to 8 wt.-%, based in the weight of the aqueous monomer solution.

In process step (iii) of the process according to the present invention a polymerization initiator or at least one component of a polymerization initiator system that comprises two or more components is added to the aqueous monomer solution.

As polymerization initiators for initiation of the polymerisation all initiators forming radicals under the polymerisation conditions can be used, which are commonly used in the production of superabsorbers. Among these belong thermal catalysts, redox catalysts and photo-initiators, whose activation occurs by energetic irradiation. The polymerisation initiators may be dissolved or dispersed in the aqueous monomer solution. The use of water-soluble catalysts is preferred.

As thermal initiators may be used all compounds known to the person skilled in the art that decompose under the effect of temperature to form radicals. Particularly preferred are thermal polymerisation initiators with a half life of less than 10 seconds, more preferably less than 5 seconds at less than 180°C, more preferably at less than 140°C. Peroxides, hydroperoxides, hydrogen peroxide, persulfates and azo compounds are particularly preferred thermal polymerisation initiators. In some cases it is advantageous to use mixtures of various thermal polymerisation initiators. Among such mixtures, those consisting of hydrogen peroxide and sodium or potassium peroxodisulfate are preferred, which may be used in any desired quantitative ratio. Suitable organic peroxides are preferably acetylacetone peroxide, methyl ethyl ketone peroxide, benzoyl peroxide, lauroyl peroxide, acetyl peroxide, capryl peroxide, isopropyl peroxidicarbonate,2-ethylhexyle peroxidicarbonate, tert.-butyl hydroperoxide, cumene hydroperoxide, tert.-amyl perpivalate, tert.-butyl perpivalate, tert.-butyl perneohexonate, tert.-butyl isobutyrate, tert.-butyl per-2-ethylhexenoate, tert.-butyl perisononanoate, tert.-butyl permaleate, tert.-butyl perbenzoate, tert.-butyl-3,5,5-trimethylhexanoate and amyl perneodecanoate. Furthermore, the following thermal polymerisation initiators are preferred: azo compounds such as azo-bis-isobutyronitrol, azo-bis-dimethylvaleronitril, 2,2-azobis-(2-amidinopropane)dihydrochloride, azo-bis-amidinopropane dihydrochloride, 2,2'-azobis-(N,N-dimethylene)isobutyramidine dihydrochloride, 2-(carbamoylazo)isobutyronitrile and 4,4'-azobis-(4-cyano-valeric acid). The aforementioned compounds are used in conventional amounts, preferably in a range from 0.01 to 5, more preferably 0.1 to 2 mol%, respectively based upon the amount of the monomers to be polymerised.

Redox catalyst comprise two of more components, usually one or more of the peroxo compounds listed above, and at least one reducing component, preferably ascorbic acid, glucose, sorbose, mannose, ammonium or alkali metal hydrogen sulfite, sulfate, thiosulfate, hyposulfite or sulfide, metal salts such as iron II ions or silver ions or sodium hydroxymethyl sulfoxylate. Preferably ascorbic acid or sodium pyrosulfite is used as reducing component of the redox catalyst. 1 × 10⁻⁵ to 1 mol % of the reducing component of the redox catalyst and 1 × 10⁻⁵ to 5 mol% of the oxidising component of the redox catalyst are used, in each case referred to the amount of monomers used in the polymerisation. Instead of the oxidising component of the redox catalyst, or as a complement thereto, one or more, preferably water-soluble azo compounds may be used.

If the polymerisation is initiated by action of energetic beams, so-called photo-initiators are generally used as initiator. These can comprise for example so-called α-splitters, H-abstracting systems or also azides. Examples of such initiators are benzophenone derivatives such as Michlers ketone, phenanthrene derivatives, fluorine derivatives, anthraquinone derivatives, thioxanthone derivatives, cumarin derivatives, benzoinether and derivatives thereof, azo compounds such as the above-mentioned radical formers, substituted hexaarylbisimidazoles or acylphosphine oxides. Examples of azides are: 2-(N,N-dimethylamino)ethyl-4-azidocinnamate, 2-(N,N-dimethylamino)ethyl-4-azidonaphthylketone, 2-(N,N-di-methylamino)ethyl-4-azidobenzoate, 5-azido-1-naphthyl-2'-(N,N-dimethylamino)ethylsulfone, N-(4-sulfonylazidophenyl)maleinimide, N-acetyl-4-sulfonylazidoaniline, 4-sulfonylazidoaniline, 4-azidoaniline, 4-azidophenacyl bromide, p-azidobenzoic acid, 2,6-bis(p-azidobenzylidene)cyclohexanone and 2,6-bis(p-azidobenzylidene)-4-methylcyclohexanone. The photo-initiators, when used, are generally employed in quantities from 0.01 to 5 wt.-% based on the monomers to be polymerised.

A particularly preferred redox system that is used in the process according to the present invention is a redox system comprising hydrogen peroxide, sodium peroxodisulfate and ascorbic acid.

In this context it should also be noted that the polymerization initiator may be added before, during or after process step (iv), i. e. after the oxygen content of the aqueous monomer solution has been decreased. If a polymerisation initiator system is used that comprises two or more components, like the preferred initiator system that comprises hydrogen peroxide, sodium peroxodisulfate and ascorbic acid and that is active only if all the components have been added, one or more of the components of such a polymerisation initiator system may, for example, be added before process step (iv), whereas the remaining component or the remaining components which are necessary to complete the activity of the polymerisation initiator system, are added after process step (iv), perhaps even after process step (v).

In process step (iv) of the process according to the present invention the oxygen content of the aqueous monomer solution is decreased, whereby it should be mentioned that process step (iv) can also be performed before, during or after process step (ii). Preferably, the oxygen content of the aqueous monomer solution is decreased after the fine particles have been added in process step (ii).

The oxygen content of the aqueous monomer solution is decreased by bringing the aqueous monomer solution into contact with an inert gas, such as nitrogen. The phase of the inert gas being in contact with the aqueous monomer solution is free of oxygen and is thus characterized by a very low oxygen partial pressure. As a consequence oxygen converts from the aqueous monomer solution into the phase of the inert gas until the oxygen partial pressures in the phase of the inert gas and the aqueous monomer solution are equal. Bringing the aqueous monomer phase into contact with a phase of an inert gas can be accomplished, for example, by introducing bubbles of the inert gas into the monomer solution in cocurrent, countercurrent or intermediate angles of entry. Good mixing can be achieved, for example, with nozzles, static or dynamic mixers or bubble columns. The oxygen content of the monomer solution before the polymerization is preferably lowered to less than 1 ppm by weight, more preferably to less than 0.5 ppm by weight.

In process step (v) of the process according to the present invention the aqueous monomer solution is charged into a polymerization reactor, preferably onto a conveyor belt, especially preferred at an upstream position of the conveyor belt and in process step (vi) the monomers in the aqueous monomer solution are polymerized in the polymerization reactor, thereby obtaining a polymer gel. If polymerization is performed on a polymerization belt as the polymerization reactor, a polymer gel strand is obtained in a downstream portion of the conveyor belt, which, before drying, is preferably comminuted in order to obtain gel particles.

As the polymerization reactor every reactor can be used which the person skilled in the art would regard as appropriate for the continuous or batchwise polymerization of monomers like acrylic acid in aqueous solutions. An example of a suitable polymerization reactor is a kneading reactor. In a kneader the polymer gel formed in the polymerization of the aqueous monomer solution is comminuted continuously by, for example, contrarotatory stirrer shafts, as described in WO 2001/38402.

Another example of a preferred polymerization reactor is a conveyor belt. As a conveyor belt that is useful for the process according to the present invention any conveyor belt can be used which the person skilled in the art considers to be useful as a support material onto which the above described aqueous monomer solution can be charged and subsequently polymerized to form a hydrogel. Examples of conveyor belts which can be used in the process according to the present invention are disclosed in DE-A-35 44 770, EP-A-0 955 086 and EP-A-1 683 813.

The conveyor belt usually comprises an endless moving conveyor belt passing over supporting elements and at least two guide rollers, of which at least one is driven and one is configured so as to be adjustable. Optionally, a winding and feed system for a release sheet that may be used in sections on the upper surface of the conveyor belt is provided. The system includes a supply and metering system for the reaction components, and optional irradiating means arranged in the direction of movement of the conveyor belt after the supply and metering system, together with cooling and heating devices, and a removal system for the polymer gel strand that is arranged in the vicinity of the guide roller for the return run of the conveyor belt. In order to provide for the completion of polymerization with the highest possible space-time yield, according to the present invention, in the vicinity of the upper run of the conveyor belt on both sides of the horizontal supporting elements, starting in the area of the supply and metering systems, there are upwardly extending supporting elements, the longitudinal axes of which intersect at a point that is beneath the upper run, and which shape the conveyor belt that is supported by them so that it become suitably trough-shaped. Thus, according to the present invention, the conveyor belt is supported in the vicinity of the supply system for the reaction components by a plurality of trough-shaped supporting and bearing elements that form a deep trough-like or dish-like configuration for the reaction components that are introduced. The desired trough-like shape is determined by the shape and arrangement of the supporting elements along the length of the path of the upper run. In the area where the reaction components are introduced, the supporting elements should be relatively close to each other, whereas in the subsequent area, after the polymerization has been initiated, the supporting elements can be arranged somewhat further apart. Both the angle of inclination of the supporting elements and the cross-section of the supporting elements can be varied in order to flatten out the initially deep trough towards the end of the polymerization section and once again bring it to an extended state. In a further embodiment of the invention, each supporting element is preferably formed by a cylindrical or spherical roller that is rotatable about its longitudinal axis. By varying both the cross-section of the roller as well as the configuration of the roller it is easy to achieve the desired cross-sectional shape of the trough. In order to ensure proper formation of the trough by the conveyor belt, both when it makes the transition from a flat to a trough-like shape and when it is once again returned to the flat shape, a conveyor belt that is flexible in both the longitudinal and the transverse directions is preferred.

In process step (vii) of the process according to the present invention the particulate polymer gel that is obtained in the polymerization reactor, preferably the polymer gel particles obtained in the kneading reactor or the polymer gel strand obtained in the downstream portion of the conveyor belt, is/are discharged out of the reactor and is/are, especially in the case of the polymer gel strand obtained on the conveyor belt, (further) comminuted, thereby obtaining polymer gel particles. Preferably, the resulting polymer gel strand is removed from the conveyor belt as a continuous strand that is of a soft semi-solid consistency and is then passed on for further processing such as comminution.

Comminution of the polymer gel strand is preferably performed in at least three steps:
- in a first step, a cutting unit, preferably a knife, for example a knife as disclosed in WO-A-96/36464, is used for cutting the polymer gel strand into flat gel strips, preferably with a length within the range of 5 to 500 mm, preferably from 10 to 300 mm and particularly preferably from 100 to 200 mm, a height within the range from 1 to 30 mm, preferably from 5 to 25 mm and particularly preferably from 10 to 20 mm as well as a width within the range from 1 to 500 mm, preferably from 5 to 250 mm and particularly preferably from 10 to 200 mm;
- in a second step, a shredding unit, preferably a breaker, is used for shredding the gel strips into gel pieces, preferably with a length within the range of 2.5 to 25 mm, preferably from 1 to 12.5 mm, a height within the range from 0.5 to 15 mm, preferably from 0.25 to 7.5 mm as well as a width within the range from 0.5 to 20 mm, preferably from 0.25 to 10 mm and
- in a third step a "wolf" (grinding) unit, preferably a wolf, preferably having a screw and a hole plate, whereby the screw conveys against the hole plate is used in order to grind and crush gel pieces into polymer gel particles which are preferably smaller than the gel pieces.

An optimal surface-volume ratio is achieved hereby, which has an advantageous effect on the drying behaviour in process step (viii). A gel which has been comminuted in this way is particularly suited to belt drying. The three-step comminution offers a better "*airability*" because of the air channels located between the granulate kernels.

In process step (viii) of the process according to the present invention the polymer gel particles are dried.

The drying of the polymer gel particles can be effected in any dryer or oven the person skilled in the art considers as appropriate for drying the above described gel particles. Rotary tube furnaces, fluidised bed dryers, plate dryers, paddle dryers and infrared dryers may be mentioned by way of example.

Especially preferred are belt driers. A belt dryer is a convective system of drying, for the particularly gentle treatment of through-airable products. The product to be dried is placed onto an endless conveyor belt which lets gas through, and is subjected to the flow of a heated gas stream, preferably air. The drying gas is recirculated in order that it may become very highly saturated in the course of repeated passage through the product layer. A certain fraction of the drying gas, preferably not less than 10 %, more preferably not less than 15 % and most preferably not less than 20 % and preferably up to 50 %, more preferably up to 40 % and most preferably up to 30 % of the gas quantity per pass, leaves the dryer as a highly saturated vapor and carries off the water quantity evaporated from the product. The temperature of the heated gas stream is preferably not less than 50°C, more preferably not less than 100°C and most preferably not less than 150°C and preferably up to 250°C, more preferably up to 220°C and most preferably up to 200°C.

The size and design of the dryers depends on the product to be processed, the manufacturing capacity and the drying duty. A belt dryer can be embodied as a single-belt, multi-belt, multi-stage or multistory system. The present invention is preferably practiced using a belt dryer having at least one belt. One-belt dryers are very particularly preferred. To ensure optimum performance of the belt-drying operation, the drying properties of the water-absorbent polymers are individually determined as a function of the processing parameters chosen. The hole size and mesh size of the belt is conformed to the product. Similarly, certain surface enhancements, such as electropolishing or Teflonizing, are possible.

The polymer gel particles to be dried are preferably applied to the belt of the belt dryer by means of a swivel belt. The feed height, i.e., the vertical distance between the swivel belt and the belt of the belt dryer, is preferably not less than 10 cm, more preferably not less than 20 cm and most preferably not less than 30 cm and preferably up to 200 cm, more preferably up to 120 cm and most preferably up to 40 cm. The thickness on the belt dryer of the polymer gel particles to be dried is preferably not less than 2 cm, more preferably not less than 5 cm and most preferably not less than 8 cm and preferably not more than 20 cm, more preferably not more than 15 cm and most preferably not more than 12 cm. The belt speed of the belt dryer is preferably not less than 0.005 m/s, more preferably not less than 0.01 m/s and most preferably not less than 0.015 m/s and preferably up to 0.05 m/s, more preferably up to 0.03 m/s and most preferably up to 0.025 m/s.

Furthermore, it is preferable according to the invention that the polymer gel particles are dried to a water content of from 0.5-25 wt.-%, preferably 1-10 wt.-% and particularly preferably 3-7 wt.-%.

In process step (ix) of the process according to the present invention the dried polymer gel particles are ground thereby obtaining particulate water-absorbent polymer particles.

For grinding of the dried polymer gel particles any device can be used the person skilled in the art considers as appropriate for grinding the above described dried polymer particles. As an example for a suitable grinding device a single- or multistage roll mill, preferably a two- or three-stage roll mill, a pin mill, a hammer mill or a vibratory mill may be mentioned.

In process step (x) of the process according to the present invention the ground water-absorbent polymer particles are sized, preferably using appropriate sieves. In this context it is particularly preferred that after sizing the water-absorbent polymer particles the content of polymer particles having a particle size of less than 150 µm is less than 10 wt.-%, preferably less than 8 wt.-% and particularly less than 6 wt.-% and that the content of polymer particles having a particle size of more than 850 µm is also less than 10 wt.-%, preferably less than 8 wt.-% and particularly less than 6 wt.-%. It is also preferred that after sizing the water-absorbent polymer particles at least 30 wt.-%, more preferred at least 40 wt.-% and most preferred at least 50 wt.-%, based on the total weight of the water-absorbent polymer particles, of the particles have a particle size in a range of from 300 to 600 µm.

In process step (xi) of the process according to the present invention the surface of the ground and sized water-absorbent polymer particles are treated. As measures to treat the surface of water-absorbent polymer particles any measure can be used the person skilled in the art considers as appropriate for such a purpose. Examples of surface treatments include, for example, surface crosslinking, the treatment of the surface with water-soluble salts, such as aluminium sulfate or aluminium lactate, the treatment of the surface with inorganic particles, such as silicon dioxide, and the like. Preferably, the components used to treat the surface of the polymer particles (cross-linker, water soluble salts) are added in the form of aqueous solutions to the water-absorbent polymer particles. After the particles have been mixed with the aqueous solutions, they are heated to a temperature in the range from 150 to 230°C, preferably 160 to 200°C in order to promote the surface-crosslinking reaction.

The process according to the present invention is characterized by the fact that in process step (i) the preparation of the aqueous monomer solution comprises the step of:
(ia) at least partially neutralizing acrylic acid by mixing acrylic acid with an aqueous solution of sodium hydroxide, thereby obtaining an aqueous solution of the at least partially neutralized acrylic acid;
(ib) cooling the aqueous solution of the at least partially neutralized acrylic acid with a cooling liquid in a heat exchanger, wherein the cooling liquid used in the heat exchanger has been cooled in an absorption chiller, wherein at least a part of the heat of the heated cooling liquid obtained in the heat exchanger is consumed by the evaporation of a refrigerant liquid in a vacuum that is created by the absorption of a refrigerant vapour into an absorbent.

In process step (ia) acrylic acid is at least partially neutralized by mixing acrylic acid with an aqueous solution of sodium hydroxide, thereby obtaining an aqueous solution of the at least partially neutralized acrylic acid. Mixing of the two components (acrylic acid and aqueous solution of sodium hydroxide) can be performed in any mixing apparatus and in any manner the person skilled in the art would consider as appropriate. The acrylic acid can be added to the aqueous solution of sodium hydroxide or an aqueous solution of sodium hydroxide can be added to the acrylic acid. Preferably the acrylic acid is added to the aqueous solution, preferably in a continuous manner wherein a stream of acrylic acid in continuously introduced into a stream of an aqueous solution of sodium hydroxide.

The temperature of the acrylic acid used in process step (ia) is preferably in the range from 15 to 35°C, more preferably in the range from 15 to 20°C.

The sodium hydroxide concentration in the aqueous solution of sodium hydroxide used in process step (ia) is preferably in the range from 10 to 30 wt.-%, more preferably in the range from 12 to 18 wt.-%, based on the total weight of the aqueous sodium hydroxide solution. The temperature of the aqueous solution of sodium hydroxide used in process step (ia) is preferably in the range from 25 to 45°C, more preferably in the range from 30 to 40°C.

As the aqueous solution of sodium hydroxide comes into contact with the acrylic acid, the energy is formed as the neutralization is a strong exothermic reaction. Without cooling the mixture the temperature would significantly rise, which would lead to the formation of impurities based on radical polymerization reaction. Therefore, the mixture obtained in process step (ia) has to be cooled.

In process step (ib) of the process according to the present invention the aqueous solution of the at least partially neutralized acrylic acid obtained in process step (ia) is cooled with a cooling liquid in a heat exchanger, wherein the cooling liquid used in the heat exchanger has been cooled in an absorption chiller wherein at least a part of the heat of the heated cooling liquid obtained in the heat exchanger (2) is consumed by the evaporation of a refrigerant liquid in a vacuum that is created by the absorption of a refrigerant vapour into an absorbent.

As a heat exchanger every apparatus can be used that enable a heat transfer from the neutralization mixture obtained in process step (ia) into a cooling liquid without requiring a direct contact between the neutralization mixture and the cooling liquid (water). Suitable heat exchanger are, for example, shell and tube heat exchangers, plate heat exchangers, adiabatic wheel heat exchangers, plate fin heat exchangers, pillow plate heat exchangers, fluid heat exchangers or phase change heat exchangers, whereby shell and tube heat exchangers and plate heat exchangers are especially preferred. The construction and function of suitable heat exchangers is described in detail in paragraph 8.1, pages 496-506 in "Grundoperationen Chemischer Verjahrenstechnik", Wilhlem R.A. Vauck, Hermann A. Müssler, Wiley-VCH, 11th edition (2000).

Preferred cooling liquids are water, aqueous salt solutions, glycerol or mixtures of these liquids, whereby water is particularly preferred as the cooling liquid.

The cooling liquid, preferably the water that is used in the heat exchanger is a cooling liquid, preferably the water that has been cooled in an absorption chiller. An absorption chiller (also called "*absorption refrigerator*") is a refrigerator that uses a heat source to provide the energy needed to drive the cooling system. The function of an absorption chiller is based on the strong affinity between the refrigerant and the absorbent used in the absorption chiller. Preferred according to the present invention is a lithium salt based absorption chiller using water as the refrigerant and a lithium salt, preferably LiBr, as the absorbent. Such absorption chillers can be obtained under the trademark "Millennium™" York International, obtainable via Johnson Controls Inc., like model YIA.

The absorption chiller that is preferred for the process according to the present invention comprises a lower shell that comprises an absorber section and an evaporator section, and an upper shell that comprises a generator section and a condenser section, wherein the pressure in the lower shell is in the range from 1 to 25 mbar, preferably in the range from 6 to 10 mbar and the pressure in the upper shell is in the range from 50 to 150 mbar, preferably in the range from 75 to 125 mbar.

In such an absorption refrigerator having so called "*natural circulation type*" the aqueous solution of the lithium salt is heated to boil in the generator section and is lifted through a gas-liquid lift into the separator located at an upper level according to the principle of an air lift pump, and is separated into water vapor and the residual concentrated lithium salt solution in the separator. This water vapor is subsequently cooled to condense in the condenser section, and the condensate or water is fed to the evaporator section. The water fed to the evaporator section is vaporized again since the internal pressure of the evaporator section is sufficiently low to such an extent that the water can be readily vaporized. Due to the fact that vaporization of the water takes heat and thus produces cooling energy, another fluid flowing through the evaporator section (= heated cooling liquid, preferably heated water from the heat exchanger used to cool the neutralization mixture) is cooled and this cooled fluid is used to cool the neutralization mixture in the heat exchanger.

In the meantime, the concentrated lithium salt solution separated from the water vapor in the separator section is supplied to a heat exchanger to be subject to heat exchange with a dilute lithium salt solution, and the concentrated lithium salt solution thus cooled down to a low temperature is then fed to the absorber section.

In the absorber section, this concentrated lithium salt solution absorbs the water vapor produced in the evaporator section to turn into a dilute lithium salt solution. This dilute lithium salt solution is subsequently fed to the heat exchanger to be subject to heat exchange with the concentrated lithium salt solution as above described, and the dilute lithium salt solution thus heated as a result of the heat exchange is returned to the generator section again. The cycle above described is repeated to carry out the desired refrigeration.

According to a preferred embodiment of the process according to the present invention energy that is generated in any of the process steps (i) through (xi), preferably energy in the form of hot water or steam that are generated in any of the process steps (i) through (xi), is/are used to operate the absorption chiller, preferably to heat the diluted absorbent solution, particularly preferred the diluted lithium salt solution that is obtained in the absorber section after the diluted absorbent solution is transferred into the generator section. Hot water that may be generated in any of the process steps (i) through (xi) is preferably a hot aqueous condensate having a temperature in the range from 180 to 240°C, preferably in the range from 200 to 220°C at a pressure in the range from 5 to 25 bar. In the process according to the present invention this hot condensate can directly be used to heat the diluted absorbent solution in the absorption chiller. However, it is also possible to produce steam out of this hot condensate and to use this steam in order to heat the diluted absorbent solution in the absorption chiller.

In the absorption chiller a cooling liquid, preferably cooling water, is used to condense the water in the condenser section and to remove the heat that is generated by the absorption process in the absorber section. Preferably, this cooling liquid is water that has been cooled by air, preferably in the cooling tower that is described below.

According to a first variant of this preferred embodiment of the process according to the present invention hot water or steam that is generated in process step (viii) is used to heat the diluted absorbent solution, particularly preferred the diluted lithium salt solution that is obtained in the absorber section after the diluted absorbent solution is transferred into the generator section. In process step (viii) the polymer gel particles obtained in process step (vii) are dried. By this dyring process large amounts of hot water or steam are generated which can be used to operate the absorption chiller in process step (ib).

According to a second variant of this preferred embodiment of the process according to the present invention hot water or steam that is generated in process step (xi) is used to heat the diluted absorbent solution, particularly preferred the diluted lithium salt solution that is obtained in the absorber section after the diluted absorbent solution is transferred into the generator section. In process step (xi) the grinded and sized water-absorbent polymer particles obtained in process step (x) are surface treated, preferably by mixing these particles with aqueous solutions comprising a crosslinking agent and optionally further additives like inorganic salts, followed by a heat-treatment to promote the surface crosslinking reaction. By this heat-treatment step also large amounts of hot water or steam are generated which can be used to operate the absorption chiller in process step (ib).

According to a third variant of this preferred embodiment of the process according to the present invention hot water or steam that is generated in process steps (viii) and (xi) is used to heat the diluted absorbent solution, particularly preferred the diluted lithium salt solution that is obtained in the absorber section after the diluted absorbent solution is transferred into the generator section.

In the process according to the present invention it has turned out to be advantageous if the neutralization mixture obtained in process step (ia) is not directly cooled in a heat exchanger that is cooled with the chilled cooling liquid, preferably with the chilled water from the absorption chiller, but if the neutralization mixture is pre-cooled in a heat exchanger that is cooled with a cooling liquid, preferably with water that has been cooled by means of other cooling devices such as a cooling tower. Accordingly, in another preferred embodiment of the process according to the present invention the aqueous solution of the at least partially neutralized acrylic acid obtained in process step (ia) is cooled in two subsequent steps:
(ib_1) cooling the aqueous solution of the at least partially neutralized acrylic acid with water in a first heat exchanger, wherein the cooling liquid, preferably the water used in the first heat exchanger as been cooled with air, preferably in a cooling tower, thereby obtaining a pre-cooled aqueous solution of the at least partially neutralized acrylic acid;
(ib_2) cooling the pre-cooled aqueous solution of the at least partially neutralized acrylic acid obtain in process step (ib_1) with a cooling liquid, preferably with water in a second heat exchanger, wherein the water used in the second heat exchanger has been cooled in an absorption chiller.

As a cooling tower every apparatus can be used that the person skilled in the art regards as appropriate for cooling the hot aqueous solution of the at least partially neutralized acrylic acid solution obtained in process step (ia) by evaporation. In this context reference is made to paragraph 8.2, pages 520-523 in "Grundoperationen Chemischer Verfahrenstechnik", Wilhlem R.A. Vauck, Hermann A. Müssler, Wiley-VCH, 11th edition (2000) where suitable cooling towers are described.

In this context it is particularly preferred that aqueous solution of the at least partially neutralized acrylic acid obtained in process step (ia) has a temperature in the range from 75 to 95°C, preferably in the range from 80 to 90°C and wherein the pre-cooled aqueous solution of the at least partially neutralized acrylic acid obtained in process step (ib_1) has a temperature in the range from 25 and 45°C, preferably in the range from 30 to 40°C.

It is furthermore preferred that the aqueous solution of the at least partially neutralized acrylic acid obtained in process step (ia) has a temperature in the range from 75 to 95°C, preferably in the range from 80 to 90°C and that the cooled aqueous solution of the at least partially neutralized acrylic acid obtained in process step (ib_2) has at temperature in the range from 10 to 40°C, preferably in the range from 12 to 35°C.

In the process according to the present invention it has also turned out to be advantageous if the acrylic acid that is used for the preparation of the water-absorbent polymer particles is not mixed with the aqueous solution of sodium hydroxide all at once, but if two or more portions of acrylic acid are subsequently added to the aqueous solution of sodium hydroxide, thereby lowering the energy that is generated by the exothermic neutralization reaction. Accordingly, in another preferred embodiment of the process according to the present invention process step (i) the preparation of the aqueous monomer solution comprises the step of:
(ia1) at least partially neutralizing acrylic acid by mixing a first portion of acrylic acid with an aqueous solution of sodium hydroxide, thereby obtaining a first aqueous solution of the at least partially neutralized acrylic acid;
(ib1) cooling the first aqueous solution of the at least partially neutralized acrylic acid with a cooling liquid, preferably with water in a heat exchanger, wherein the cooling liquid used in the heat exchanger has been cooled in an absorption chiller.
(ia2) at least partially neutralizing acrylic acid by mixing a second portion of acrylic acid with the cooled first aqueous solution of the at least partially neutralized acrylic acid that has been obtained in process step (ib1), thereby obtaining a second aqueous solution of the at least partially neutralized acrylic acid;
(ib2) cooling the second aqueous solution of the at least partially neutralized acrylic acid with a cooling liquid, preferably with in a heat exchanger, wherein the cooling liquid used in the heat exchanger has been cooled in an absorption chiller.

In this context it is particularly preferred that in process step (ia1) between 50 and 90 wt.-%, more preferably between 60 and 80 wt.-% of the total amount of acrylic acid that is present in the monomer solution being polymerized in process step (iv) is added to the aqueous solution of sodium hydroxide as the first portion, the remainder being added as the second portion in process step (ia2).

Again, the cooling of the aqueous solution of the at least partially neutralized acrylic acid in process steps (ib1) and (ib2) can again be performed in two steps (ib1_1 and (ib1_2) or (ib2_1 and ib2_2), respectively) wherein the aqueous solutions of the at least partially neutralized acrylic acid obtained in process steps (ia1) and (ia2), respectively, are cooled with water in a first heat exchanger, wherein the cooling liquid, preferably the water used in the first heat exchanger as been cooled with air, preferably in a cooling tower, thereby obtaining pre-cooled aqueous solutions of the at least partially neutralized acrylic acid, and wherein these pre-cooled aqueous solutions of the at least partially neutralized acrylic acid are further cooled with a cooling liquid, preferably with water, in a second heat exchanger, wherein the water used in the second heat exchanger has been cooled in an absorption chiller.

A contribution to the solution of the objects mentioned at the beginning is also made by water-absorbent polymer particles which are obtainable by such a process.

A further contribution to achieving the objects described at the beginning is made by a composite material comprising the water-absorbent polymer particles obtainable by the process according to the present invention and a substrate. In this context, it is preferable for the water-absorbent polymer particles and the substrate to be firmly bonded to one another. Preferred substrates are films of polymers, such as, for example, of polyethylene, polypropylene or polyamide, metals, nonwovens, fluff, tissues, woven fabric, natural or synthetic fibres, or other foams. It is furthermore preferable for the composite material to include at least one region which comprises the water-absorbent polymer particles in an amount in the range of from about 15 to 100 wt.%, preferably about 30 to 100 wt.%, particularly preferably from about 50 to 99.99 wt.%, furthermore preferably from about 60 to 99.99 wt.% and moreover preferably from about 70 to 99 wt.%, in each case based on the total weight of the composite material region in question, this region preferably having a size of at least 0.01 cm³, preferably at least 0.1 cm³ and most preferably at least 0.5 cm³.

In a particularly preferred embodiment of the composite material, this is a planar composite material such as is described as "absorbent material" in WO 02/056812 A1. The WO 02/056812 A1 disloses in particular with respect to the precise structure of the composite material, the weight per unit area of its constituents and its thickness.

A further contribution to achieving the objects mentioned at the beginning is made by a process for the production of a composite material, wherein the water-absorbent polymer particles obtainable by the process according to the present invention and a substrate and optionally an additive are brought into contact with one another. Substrates which are employed are preferably those substrates which have already been mentioned above in connection with the composite material.

A contribution to achieving the objects mentioned at the beginning is also made by a composite material obtainable by the process described above, this composite material preferably having the same properties as the composite material described above.

A further contribution to achieving the objects mentioned at the beginning is made by chemical products comprising the water-absorbent polymer particles obtainable by the process according to the present invention or a composite material. Preferred chemical products are, in particular, foams, shaped articles, fibres, foils, films, cables, sealing materials, liquid-absorbing hygiene articles, in particular nappies and sanitary towels, carriers for plant or fungal growth-regulating agents or plant protection active compounds, additives for building materials, packaging materials or soil additives.

The use of the water-absorbent polymer particles obtainable by the process according to the present invention or of the composite material in chemical products, preferably in the abovementioned chemical products, in particular in hygiene articles, such as nappies or sanitary towels, and the use of the superabsorber particles as carriers for plant or fungal growth-regulating agents or plant protection active compounds also make a contribution to achieving the abovementioned objects. In the use as a carrier for plant or fungal growth-regulating agents or plant protection active compounds, it is preferable for the plant or fungal growth-regulating agents or plant protection active compounds to be able to be released over a period of time controlled by the carrier.

The invention is now more closely illustrated by non-limiting figures.
Figure 1 is a schematic diagram of the basic principle of the process according to the present invention.
Figure 2 illustrates the mode of action of an absorption chiller.
Figure 3 is a schematic diagram of a preferred embodiment of the process according to the present invention wherein steam that is generated in the course of the preparation of the water-absorbent polymer particles is used to operate the absorption chiller.
Figure 4 illustrates preferred sources of steam being generated in the course of the preparation of the water-absorbent polymer particles and being used to operate the absorption chiller.
Figure 5 is a schematic diagram of another preferred embodiment of the process according to the present invention wherein acrylic acid is introduced into the process in two portions.

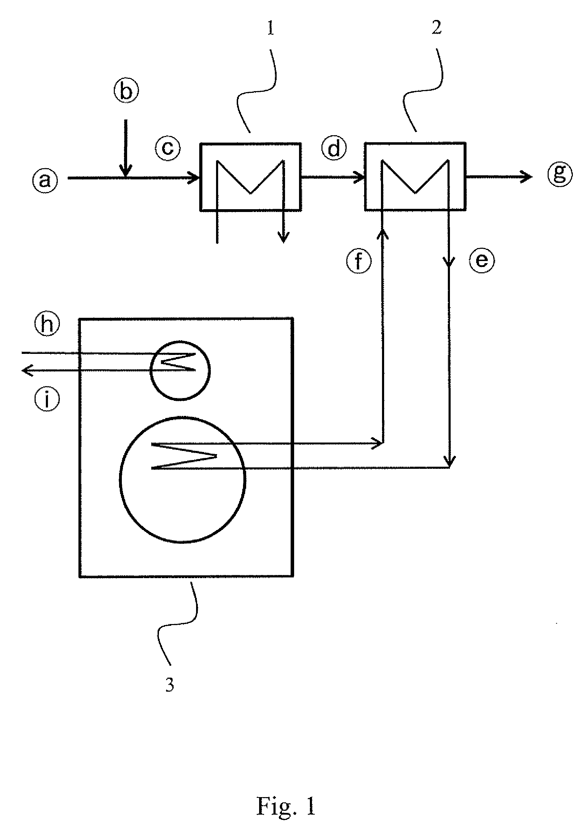

Figure 1 is a schematic diagram of the basic principle of the process according to the present invention. A stream of an aqueous solution of sodium hydroxide ⓐ is combined with a stream of acrylic acid ⓑ according to process step (ia). The thus obtained mixture ⓒ is pre-cooled in a first heat exchanger 1. The hot coolant (water) of the first heat exchanger 1 is preferably cooled in a cooling tower (not shown). The temperature of the stream ⓓ of the aqueous solution of partially neutralized acrylic acid after it has passed the first heat exchanger 1 is preferably between 30 and 40°C. Stream ⓓ is than further cooled in a second heat exchanger 2. The coolant used to operate this second heat exchanger 2 is cooled in an absorption chiller 3. Stream ⓔ of the heated coolant of the second heat exchanger 2 is cooled in the evaporator section of the absorption chiller 3 and is recirculated as a cooled stream ⓕ into the heat exchanger 2. The absorption chiller is operated by a stream of hot water or steam ⓗ that leaves the absorption chiller as stream ⓘ. After having passed the heat exchanger 2 a stream ⓖ of an aqueous solution of partially neutralized acrylic acid is obtained having a temperature in the range from 10 to 40°C.

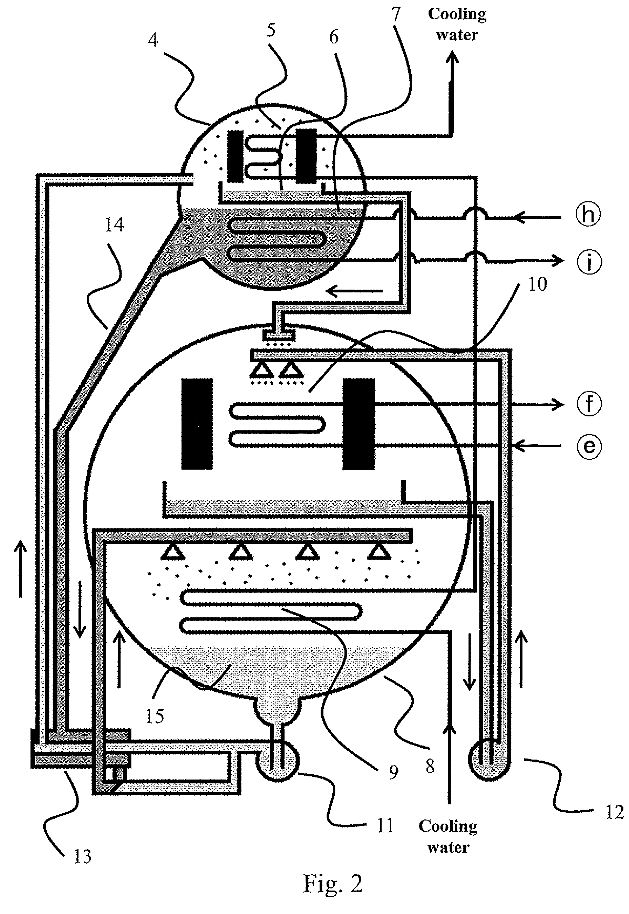

Figure 2 illustrates the mode of action of the absorption chiller 3 that uses water as the refrigerant an a lithium salt like lithium bromide as the absorbent. The absorption chiller 3 comprises a lower shell 8 that comprises an absorber section 9 and an evaporator section 10 and an upper shell 4 that comprises a generator section 7 and a condenser section 5, wherein the pressure in the lower shell 8 is about 7 mbar and the pressure in the upper section 4 is about 90 mbar. The cooling water use to remove the heat generated in the absorber section 9 and to condense the refrigerant in the condenser section 6 is preferably water that has been cooled in a cooling tower.

The aqueous solution of the lithium salt 14 (so called "*strong solution*") is heated to boil in the generator section 7 and is lifted through a gas-liquid lift into the separator located at an upper level according to the principle of an air lift pump, and is separated into water vapor and the residual concentrated lithium salt solution in the separator. This water vapor is subsequently cooled to condense in the condenser section 5, and the condensate or water 6 is fed to the evaporator section 10. The water fed to the evaporator section 10 is vaporized again since the internal pressure of the evaporator section 6 is sufficiently low to such an extent that the water can be readily vaporized. Due to the fact that vaporization of the water takes heat and thus produces cooling energy, another fluid flowing through the evaporator section (= heated water from the heat exchanger used to cool the neutralization mixture; stream ⓔ) is cooled and this cooled fluid (stream ⓕ) is used to cool the neutralization mixture in the heat exchanger 2.

In the meantime, the concentrated lithium salt solution separated from the water vapor in the separator is supplied to a heat exchanger 13 to be subject to heat exchange with a dilute lithium salt solution 15 (so called "*dilate solutions*")*,* and the concentrated lithium salt solution thus cooled down to a low temperature is then fed to the absorber section 9. In the absorber section 9, this concentrated lithium salt solution absorbs the water vapor produced in the evaporator section 10 to turn into a dilute lithium salt solution. This dilute lithium salt solution is subsequently fed to the heat exchanger 13 to be subject to heat exchange with the concentrated lithium salt solution as above described, and the dilute lithium salt solution thus heated as a result of the heat exchange is returned to the generator section 7 again. The cycle above described is repeated to carry out the desired refrigeration.

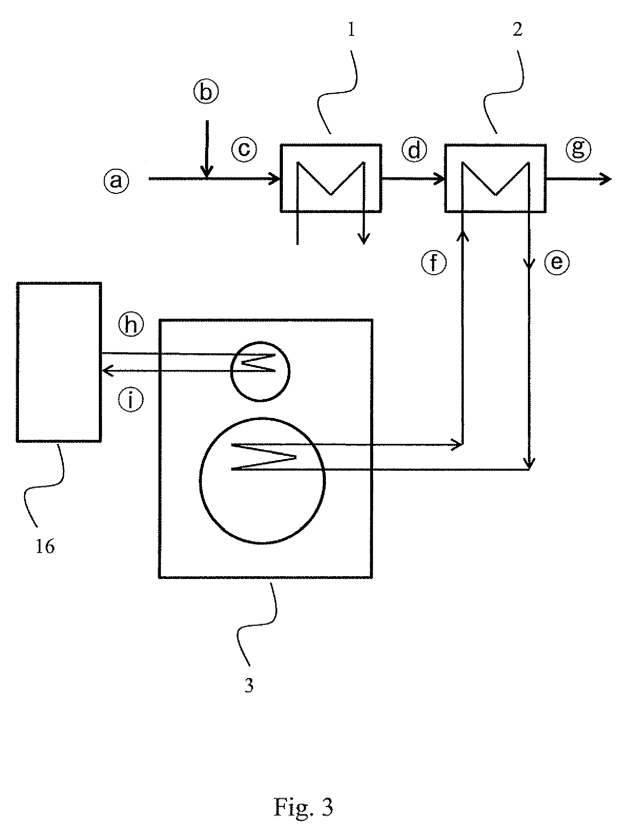

Figure 3 is a schematic diagram of a preferred embodiment of the process according to the present invention wherein steam ⓗ that is generated in the course of the preparation of the water-absorbent polymer particles in the production unit 16 is used to operate the absorption chiller 3. Steam ⓗ enters the absorption chiller 3 in the generator section 7 in the upper shell 4 (see figure 2) and heats the aqueous solution of the lithium salt 14.

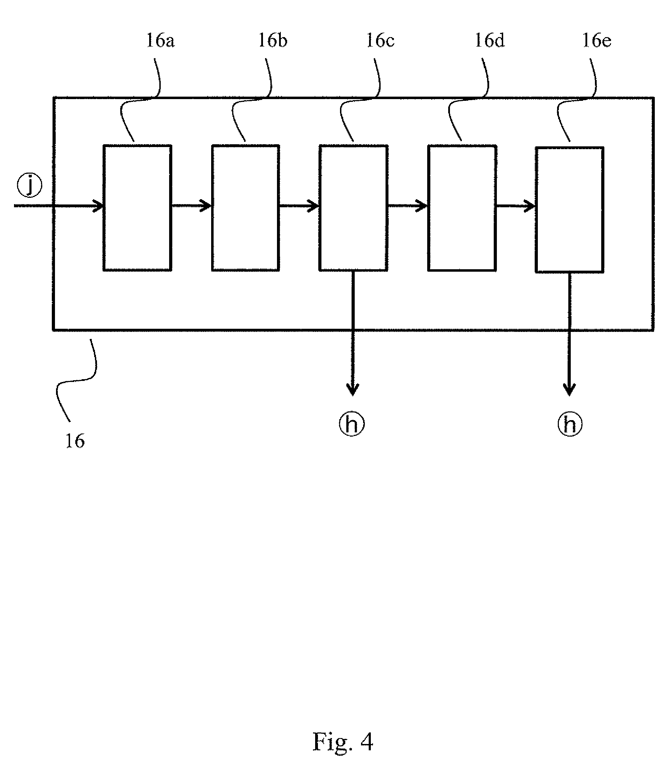

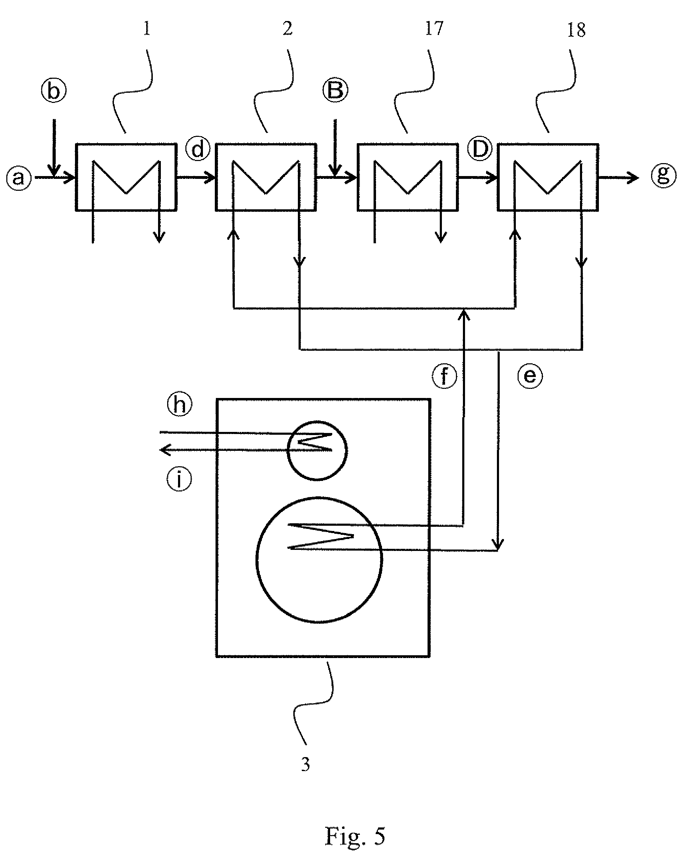

Figure 4 illustrates preferred sources of steam ⓗ being generated in the course of the preparation of the water-absorbent polymer particles and being used to operate the absorption chiller 3. In a production unit 15 for preparing water absorbent polymer particles a cooled stream ⓙ of an aqueous monomer solution is introduced. The stream preferably corresponds to stream ⓖ in figures 1 and 5. The monomer solution may be supplemented in the production unit 16 with further additives such as crosslinkers, initiators, fines etc. and is then polymerized in a polymerization unit 16a. The thus obtained polymer gel is comminuted in a comminuting unit 16b whereupon polymer gel particles are obtained. These polymer gel particles are dried in a drying unit 16c and the dried polymer is subsequently grinded and classified in a grinding- and classifying unit 16d. In surface crosslinking unit 16e the thus obtained water-absorbent polymer particles are surface crosslinked. Especially in the drying unit 16 c and in the surface crosslinking unit 16e steam ⓗ is generated as a by-product which can serve to operate the absorption chiller as illustrated in figure 2.

Figure 5 is a schematic diagram of another preferred embodiment of the process according to the present invention wherein acrylic acid is introduced into the process in two portions. A stream of an aqueous solution of sodium hydroxide ⓐ having a temperature in the range from 25 to 45°C, preferably 30 to 40°C, is combined with a first stream acrylic acid ⓑ according to process step (ia). About 70 wt.-% of the acrylic acid that is present in the final monomer mixture used to prepare the polymer is added in this process step. The thus obtained mixture having a temperature in the range from 75 to 95°C, preferably from 80 to 90°C, is pre-cooled in a first heat exchanger 1. The hot coolant (water) of the first heat exchanger 1 is preferably cooled in a cooling tower (not shown). The temperature of the stream ⓓ of the aqueous solution of partially neutralized acrylic acid after it has passed the first heat exchanger is between 25 to 45°C, preferably between 30 and 40°C. Stream ⓓ is then further cooled in a second heat exchanger 2. The coolant used to operate this second heat exchanger 2 is cooled in an absorption chiller 3. Stream ⓔ of the heated coolant of the second heat exchanger 2 is cooled in the evaporator section of the absorption chiller 3 and is recirculated as a cooled stream ⓕ into the heat exchanger 2. The absorption chiller is operated by a stream of hot water or steam ⓗ that leaves the absorption chiller as stream ⓘ. After having passed the heat exchanger 2 a stream of an aqueous solution of partially neutralized acrylic acid is obtained that has a temperature in the range from 10 to 20°C, preferably from 12 to 15°C. To this aqueous solution of partially neutralized acrylic acid the reminder portion of acrylic acid (about 30 wt.-%) is added as stream Ⓑ. The thus obtained aqueous solution of partially neutralized acrylic acid has a temperature in the range from 20 to 45°C, preferably from 30 to 35°C and is then cooled in the same way as the stream that has been obtained by mixture of streams ⓐ and ⓑ. Again, the thus obtained mixture is pre-cooled in a first heat exchanger 17. The hot coolant (water) of the first heat exchanger 17 is preferably cooled in a cooling tower (not shown). The temperature of the stream Ⓓ of the aqueous solution of partially neutralized acrylic acid after it has passed the first heat exchanger 17 is preferably between 20 and 30°C. Stream Ⓓ is then further cooled in a second heat exchanger 18. The coolant used to operate this second heat exchanger 18 is cooled in an absorption chiller 3. After having passed the heat exchanger 2 a stream of an aqueous solution of partially neutralized acrylic acid is obtained that has a temperature in the range from 10 to 40°C, preferably from 12 to 35°C.

### LIST OF REFERENCE SYMBOLS

- 1: first heat exchanger
- 2: second heat exchanger
- 3: absorption chiller (absorption refrigerator)
- 4: upper shell
- 5: condensor section
- 6: condensate (water)
- 7: generator section
- 8: lower shell
- 9: absorber section
- 10: evaporator section
- 11: pump
- 12: pump
- 13: heat exchanger
- 14: strong solution
- 15: dilute solution
- 16: production unit for the preparation of water-absorbent polymer particles
- 16a: polymerization unit
- 16b: comminuting unit
- 16c: drying unit
- 16d: grinding- and classifying unit
- 16e: surface crosslinking unit
- 17: first heat exchanger
- 18: second heat exchanger

## Claims

1. A process for the preparation of water-absorbent polymer particles, comprising the process steps of
(i) preparing an aqueous monomer solution comprising at least partially neutralized, monoethylenically unsaturated monomers bearing carboxylic acid groups (α1) and at least one crosslinker (α3);
(ii) optionally adding fine particles of a water-absorbent polymer to the aqueous monomer solution;
(iii) adding a polymerization initiator or a at least one component of a polymerization initiator system that comprises two or more components to the aqueous monomer solution;
(iv) decreasing the oxygen content of the aqueous monomer solution;
(v) charging the aqueous monomer solution into a polymerization reactor;
(vi) polymerizing the monomers in the aqueous monomer solution in the polymerization reactor thereby obtaining a polymer gel;
(vii) discharging the polymer gel out of the polymerization reactor and optionally comminuting the polymer gel thereby obtaining polymer gel particles;
(viii) drying the polymer gel particles;
(ix) grinding the dried polymer gel particles thereby obtaining particulate water-absorbent polymer particles;
(x) sizing the grinded water-absorbent polymer particles; and
(xi) treating the surface of the grinded and sized water-absorbent polymer particles;
wherein in process step (i) the preparation of the aqueous monomer solution comprises the step of:
(ia) at least partially neutralizing acrylic acid by mixing acrylic acid with an aqueous solution of sodium hydroxide, thereby obtaining an aqueous solution of the at least partially neutralized acrylic acid;
(ib) cooling the aqueous solution of the at least partially neutralized acrylic acid with a cooling liquid in a heat exchanger (2), wherein the cooling liquid used in the heat exchanger has been cooled in an absorption chiller (3) in which at least a part of the heat of the heated cooling liquid obtained in the heat exchanger (2) is consumed by the evaporation of a refrigerant liquid in a vacuum that is created by the absorption of a refrigerant vapour into an absorbent.

2. The process according to claim 1, wherein the aqueous solution of the at least partially neutralized acrylic acid obtained in process step (ia) has a temperature in the range from 75 to 95°C and wherein the aqueous solution of the at least partially neutralized acrylic acid obtained in process step (ib) has a temperature in the range from 10 to 20°C.

3. The process according to claim 1 or 2, wherein the aqueous solution of the at least partially neutralized acrylic acid obtained in process step (ia) is cooled in two subsequent steps:
(ib_1) cooling the aqueous solution of the at least partially neutralized acrylic acid with a cooling liquid in a first heat exchanger (1), wherein the cooling liquid used in the first heat exchanger (1) has been cooled with air, thereby obtaining a pre-cooled aqueous solution of the at least partially neutralized acrylic acid;
(ib_2) cooling the pre-cooled aqueous solution of the at least partially neutralized acrylic acid obtained in process step (ib_1) with a cooling liquid in a second heat exchanger (2), wherein the cooling liquid used in the second heat exchanger (2) has been cooled in an absorption chiller (3).

4. The process according to claim 3, wherein the aqueous solution of the at least partially neutralized acrylic acid obtained in process step (ia) has a temperature in the range from 75 to 95°C and wherein the pre-cooled aqueous solution of the at least partially neutralized acrylic acid obtained in process step (ib_1) has a temperature in the range from 25 to 45°C and the cooled aqueous solution of the at least partially neutralized acrylic acid obtained in process step (ib_2) has a temperature in the range from 10 to 40°C.

5. The process according to anyone of the preceding claims, wherein the absorption chiller (3) is a lithium salt based absorption chiller (3) using water as the refrigerant and a lithium salt as the absorbent.

6. The process according to anyone of the preceding claims, wherein the absorption chiller (3) comprises a lower shell (8) that comprises an absorber section (9) and an evaporator section (10), and an upper shell (4) that comprises a generator section (7) and a condenser section (5), wherein the pressure in the lower shell (8) is in the range from 1 to 25 mbar and the pressure in the upper shell (4) is in the range from 50 to 150 mbar.

7. The process according to claim 6, wherein hot water or steam that is generated in at least one of the process steps (i) through (xi) is used to operate the absorption chiller.

8. The process according to claim 7, wherein hot water or steam that is generated in process step (viii) is used to heat the diluted absorbent solution that is obtained in the absorber section (9) after the diluted absorbent solution is transferred into the generator section (7).

9. The process according to claim 7 or 8, wherein hot water or steam that is generated in process step (xi) is used to heat the diluted absorbent solution that is obtained in the absorber section (9) after the diluted absorbent solution is transferred into the generator section (7).

10. The process according to anyone of the preceding claims, wherein in process step (i) the preparation of the aqueous monomer solution comprises the step of:
(ia1) at least partially neutralizing acrylic acid by mixing a first portion of acrylic acid with an aqueous solution of sodium hydroxide, thereby obtaining a first aqueous solution of the at least partially neutralized acrylic acid;
(ib1) cooling the first aqueous solution of the at least partially neutralized acrylic acid with a cooling liquid in a heat exchanger (2), wherein the cooling liquid used in the heat exchanger (2) has been cooled in an absorption chiller (3);
(ia2) at least partially neutralizing acrylic acid by mixing a second portion of acrylic acid with the cooled first aqueous solution of the at least partially neutralized acrylic acid that has been obtained in process step (ib1), thereby obtaining a second aqueous solution of the at least partially neutralized acrylic acid;
(ib2) cooling the second aqueous solution of the at least partially neutralized acrylic acid with a cooling liquid in a heat exchanger (2), wherein the cooling liquid used in the heat exchanger (2) has been cooled in an absorption chiller (3).

11. The process according to claim 10, wherein in process step (ia1) between 50 and 90 wt.-% of the total amount of acrylic acid that is present in the monomer solution being polymerized in process step (iv) is added to the aqueous solution of sodium hydroxide as the first portion, the remainder being added as the second portion in process step (ia2).

12. The process according to claim 11, wherein in process step (ia1) between 60 and 80 wt.-% of the total amount of acrylic acid that is present in the monomer solution being polymerized in process step (iv) is added to the aqueous solution of sodium hydroxide as the first portion, the remainder being added as the second portion in process step (ia2).

13. The process according to anyone of the preceding claims, wherein the aqueous solution of sodium hydroxide used in process step (ia) has a temperature in the range from 25 to 55°C.

14. The process according to anyone of the preceding claims, wherein the aqueous solution of sodium hydroxide used in process step (ia) has a sodium hydroxide concentration in the range from 10 to 30 wt.-%, based on the total weight of the aqueous sodium hydroxide solution.

## Patentansprüche

1. Verfahren zur Herstellung wasserabsorbierender Polymerpartikel, das die folgenden Verfahrensschritte aufweist:
(i) Herstellen einer wässrigen Monomerlösung, umfassend mindestens teilweise neutralisierte, monoethylenisch ungesättigte Monomere, die Carbonsäuregruppen (α1) tragen, und mindestens ein Vernetzungsmittel (α3);
(ii) wahlweise Hinzufügen von Feinpartikeln eines wasserabsorbierenden Polymers zu der wässrigen Monomerlösung;
(iii) Hinzufügen eines Polymerisationsinitiators oder mindestens einer Komponente eines Polymerisationsinitiatorsystems, das zwei oder mehr Komponenten aufweist, zu der wässrigen Monomerlösung;
(iv) Reduzieren des Sauerstoffgehalts der wässrigen Monomerlösung;
(v) Laden der wässrigen Monomerlösung in einen Polymerisationsreaktor;
(vi) Polymerisieren der Monomere in der wässrigen Monomerlösung in dem Polymerisationsreaktor, wodurch ein Polymergel erhalten wird;
(vii) Ausleiten des Polymergels aus dem Polymerisationsreaktor und wahlweise Zerkleinern des Polymergels, wodurch Polymergelpartikel erhalten werden;
(viii) Trocknen der Polymergelpartikel;
(ix) Mahlen der getrockneten Polymergelpartikel, wodurch partikuläre wasserabsorbierende Polymerpartikel erhalten werden;
(x) Größenmessen der gemahlenen wasserabsorbierenden Polymerpartikel; und
(xi) Behandeln der Oberfläche der gemahlenen und nach Größe gemessenen wasserabsorbierenden Polymerpartikel;
wobei in Verfahrensschritt (i) die Herstellung der wässrigen Monomerlösung die folgenden Schritte aufweist:
(ia) mindestens teilweises Neutralisieren einer Acrylsäure durch Mischen der Acrylsäure mit einer wässrigen Natriumhydroxidlösung, wodurch eine wässrige Lösung der mindestens teilweise neutralisierten Acrylsäure erhalten wird;
(ib) Kühlen der wässrigen Lösung der mindestens teilweise neutralisierten Acrylsäure mit einer Kühlflüssigkeit in einem Wärmetauscher (2), wobei die in dem Wärmetauscher verwendete Kühlflüssigkeit in einer Absorptionskälteanlage (3) gekühlt worden ist, in der mindestens ein Teil der in dem Wärmetauscher (2) erhaltenen Wärme der erwärmten Kühlflüssigkeit durch die Verdunstung einer Kühlmittelflüssigkeit in einem Vakuum verbraucht wird, das durch die Absorption eines Kühlmitteldampfes in ein Absorptionsmittel erzeugt wird.

2. Verfahren nach Anspruch 1, wobei die wässrige Lösung der in Verfahrensschritt (ia) erhaltenen mindestens teilweise neutralisierten Acrylsäure eine Temperatur im Bereich von 75 bis 95 °C aufweist und wobei die wässrige Lösung der in Verfahrensschritt (ib) erhaltenen mindestens teilweise neutralisierten Acrylsäure eine Temperatur im Bereich von 10 bis 20 °C aufweist.

3. Verfahren nach Anspruch 1 oder 2, wobei die wässrige Lösung der in Verfahrensschritt (ia) erhaltenen mindestens teilweise neutralisierten Acrylsäure in zwei nachfolgenden Schritten gekühlt wird:
(ib_1) Kühlen der wässrigen Lösung der mindestens teilweise neutralisierten Acrylsäure mit einer Kühlflüssigkeit in einem ersten Wärmetauscher (1), wobei die in dem ersten Wärmetauscher (1) verwendete Kühlflüssigkeit mit Luft gekühlt worden ist, wodurch eine vorgekühlte Lösung der mindestens teilweise neutralisierten Acrylsäure erhalten wird;
(ib_2) Kühlen der in Verfahrensschritt (ib_1) erhaltenen vorgekühlten wässrigen Lösung der mindestens teilweise neutralisierten Acrylsäure mit einer Kühlflüssigkeit in einem zweiten Wärmetauscher (2), wobei die in dem zweiten Wärmetauscher (2) verwendete Kühlflüssigkeit in einer Absorptionskälteanlage (3) gekühlt worden ist.

4. Verfahren nach Anspruch 3, wobei die in Verfahrensschritt (ia) erhaltene wässrige Lösung der mindestens teilweise neutralisierten Acrylsäure eine Temperatur im Bereich von 75 bis 95 °C aufweist und wobei die in Verfahrensschritt (ib_1) erhaltene vorgekühlte wässrige Lösung der mindestens teilweise neutralisierten Acrylsäure eine Temperatur im Bereich von 25 bis 45 °C aufweist und die in Verfahrensschritt (ib_2) erhaltene gekühlte wässrige Lösung der mindestens teilweise neutralisierten Acrylsäure eine Temperatur im Bereich von 10 bis 40 °C aufweist.

5. Verfahren nach einem der vorhergehenden Ansprüche, wobei es sich bei der Absorptionskälteanlage (3) um eine Absorptionskälteanlage auf Lithiumsalzbasis (3) handelt, wobei Wasser als Kühlmittel und ein Lithiumsalz als Absorptionsmittel verwendet werden.

6. Verfahren nach einem der vorhergehenden Ansprüche, wobei die Absorptionskälteanlage (3) ein unteres Gehäuse (8) aufweist, welches einen Absorberabschnitt (9) und einen Verdunsterabschnitt (10) aufweist, und ein oberes Gehäuse (4), welches einen Generatorabschnitt (7) und einen Kondensatorabschnitt (5) aufweist, wobei der Druck in dem unteren Gehäuse (8) im Bereich von 1 bis 25 mbar liegt und der Druck in dem oberen Gehäuse (4) im Bereich von 50 bis 150 mbar liegt.

7. Verfahren nach Anspruch 6, wobei heißes Wasser oder Dampf, das bzw. der in mindestens einem der Verfahrensschritte (i) bis (xi) erzeugt wird, verwendet wird, um die Absorptionskälteanlage zu betreiben.

8. Verfahren nach Anspruch 7, wobei heißes Wasser oder Dampf, das bzw. der in Verfahrensschritt (viii) erzeugt wird, verwendet wird, um die verdünnte absorbierende Lösung, die im Absorberabschnitt (9) erhalten wird, zu erwärmen, nachdem die verdünnte absorbierende Lösung in den Generatorabschnitt (7) überführt worden ist.

9. Verfahren nach Anspruch 7 oder 8, wobei heißes Wasser oder Dampf, das bzw. der in Verfahrensschritt (xi) erzeugt wird, verwendet wird, um die verdünnte absorbierende Lösung, die im Absorberabschnitt (9) erhalten wird, zu erwärmen, nachdem die verdünnte absorbierende Lösung in den Generatorabschnitt (7) überführt worden ist.

10. Verfahren nach einem der vorhergehenden Ansprüche, wobei in Verfahrensschritt (i) die Herstellung der wässrigen Monomerlösung die folgenden Schritte aufweist:
(ia1) mindestens teilweises Neutralisieren einer Acrylsäure durch Mischen einer ersten Portion der Acrylsäure mit einer wässrigen Natriumhydroxidlösung, wodurch eine erste wässrige Lösung der mindestens teilweise neutralisierten Acrylsäure erhalten wird;
(ib1) Kühlen der ersten wässrigen Lösung der mindestens teilweise neutralisierten Acrylsäure mit einer Kühlflüssigkeit in einem Wärmetauscher (2), wobei die in dem Wärmetauscher (2) verwendete Kühlflüssigkeit in einer Absorptionskälteanlage (3) gekühlt worden ist;
(ia2) mindestens teilweises Neutralisieren einer Acrylsäure durch Mischen einer zweiten Portion der Acrylsäure mit der in Verfahrensschritt (ib1) erhaltenen gekühlten ersten wässrigen Lösung der mindestens teilweise neutralisierten Acrylsäure, wodurch eine zweite wässrige Lösung der mindestens teilweise neutralisierten Acrylsäure erhalten wird;
(ib2) Kühlen der zweiten wässrigen Lösung der mindestens teilweise neutralisierten Acrylsäure mit einer Kühlflüssigkeit in einem Wärmetauscher (2), wobei die in dem Wärmetauscher (2) verwendete Kühlflüssigkeit in einer Absorptionskälteanlage (3) gekühlt worden ist.

11. Verfahren nach Anspruch 10, wobei in Verfahrensschritt (ia1) 50 bis 90 Gew.-% der Gesamtmenge an Acrylsäure, die in der Monomerlösung vorhanden ist, welche in Verfahrensschritt (iv) polymerisiert wird, als die erste Portion der wässrigen Natriumhydroxidlösung zugegeben wird, wobei der Rest als die zweite Portion in Verfahrensschritt (ia2) zugegeben wird.

12. Verfahren nach Anspruch 11, wobei in Verfahrensschritt (ia1) 60 bis 80 Gew.-% der Gesamtmenge an Acrylsäure, die in der Monomerlösung vorhanden ist, welche in Verfahrensschritt (iv) polymerisiert wird, als die erste Portion der wässrigen Natriumhydroxidlösung zugegeben wird, wobei der Rest als die zweite Portion in Verfahrensschritt (ia2) zugegeben wird.

13. Verfahren nach einem der vorhergehenden Ansprüche, wobei die in Verfahrensschritt (ia) verwendete wässrige Natriumhydroxidlösung eine Temperatur im Bereich von 25 bis 55 °C aufweist.

14. Verfahren nach einem der vorhergehenden Ansprüche, wobei die in Verfahrensschritt (ia) verwendete wässrige Natriumhydroxidlösung basierend auf dem Gesamtgewicht der wässrigen Natriumhydroxidlösung eine Natriumhydroxidkonzentration im Bereich von 10 bis 30 Gew.-% aufweist.

## Revendications

1. Procédé pour la préparation de particules polymères absorbant l'eau, comprenant les étapes de procédé consistant à
(i) préparer une solution aqueuse de monomères comprenant des monomères à insaturation monoéthylénique au moins partiellement neutralisés, portant des groupes acide carboxylique (α1) et au moins un agent de réticulation (α3) ;
(ii) éventuellement ajouter à la solution aqueuse de monomères de fines particules d'un polymère absorbant l'eau ;
(iii) ajouter à la solution aqueuse de monomères un amorceur de polymérisation ou au moins un composant d'un système amorceur de polymérisation qui comprend deux composants ou plus ;
(iv) réduire la teneur en oxygène de la solution aqueuse de monomères ;
(v) charger la solution aqueuse de monomères dans un réacteur de polymérisation ;
(vi) polymériser les monomères dans la solution aqueuse de monomères dans le réacteur de polymérisation, ce qui donne ainsi un gel polymère ;
(vii) décharger le gel polymère hors du réacteur de polymérisation et éventuellement fragmenter le gel polymère, ce qui donne ainsi des particules de gel polymère ;
(viii) sécher les particules de gel polymère ;
(ix) broyer les particules de gel polymère séchées, ce qui donne ainsi des particules polymères absorbant l'eau particulaire ;
(x) tamiser les particules polymères absorbant l'eau broyées ; et
(xi) traiter la surface des particules polymères absorbant l'eau broyées et tamisées ;
dans lequel, dans l'étape de procédé (i), la préparation de la solution aqueuse de monomères comprend les étapes consistant à :
(ia) neutraliser au moins partiellement de l'acide acrylique en mélangeant de l'acide acrylique avec une solution aqueuse d'hydroxyde de sodium, ce qui donne ainsi une solution aqueuse de l'acide acrylique au moins partiellement neutralisé ;
(ib) refroidir la solution aqueuse de l'acide acrylique au moins partiellement neutralisé avec un liquide réfrigérant dans un échangeur de chaleur (2), le liquide réfrigérant utilisé dans l'échangeur de chaleur ayant été refroidi dans un refroidisseur par absorption (3) dans lequel au moins une partie de la chaleur du liquide de refroidissement chauffé obtenu dans l'échangeur de chaleur (2) est consommée par l'évaporation d'un liquide réfrigérant dans un vide qui est créé par l'absorption d'une vapeur de réfrigérant dans un absorbant.

2. Procédé selon la revendication 1, dans lequel la solution aqueuse de l'acide acrylique au moins partiellement neutralisé, obtenue dans l'étape de procédé (ia), a une température située dans la plage allant de 75 à 95°C, et dans lequel la solution aqueuse de l'acide acrylique au moins partiellement neutralisé, obtenue dans l'étape de procédé (ib), a une température située dans la plage allant de 10 à 20°C.

3. Procédé selon la revendication 1 ou 2, dans lequel la solution aqueuse de l'acide acrylique au moins partiellement neutralisé, obtenue dans l'étape de procédé (ia), est refroidie dans deux étapes subséquentes :
(ib-1) la solution aqueuse de l'acide acrylique au moins partiellement neutralisé est refroidie avec un liquide de refroidissement dans un premier échangeur de chaleur (1), le liquide de refroidissement utilisé dans le premier échangeur de chaleur (1) ayant été refroidi avec de l'air, ce qui donne ainsi une solution aqueuse pré-refroidie de l'acide acrylique au moins partiellement neutralisé ;
(ib-2) la solution aqueuse pré-refroidie de l'acide acrylique au moins partiellement neutralisé, obtenue dans l'étape de procédé (ib_1), est refroidie avec un liquide de refroidissement dans un deuxième échangeur de chaleur (2), le liquide de refroidissement utilisé dans le deuxième échangeur de chaleur (2) ayant été refroidi dans un refroidisseur par absorption (3).

4. Procédé selon la revendication 3, dans lequel la solution aqueuse de l'acide acrylique au moins partiellement neutralisé, obtenue dans l'étape de procédé (ia), a une température située dans la plage allant de 75 à 95°C, et dans lequel la solution aqueuse pré-refroidie de l'acide acrylique au moins partiellement neutralisé, obtenue dans l'étape de procédé (ib_1), a une température située dans la plage allant de 25 à 45°C et la solution aqueuse refroidie de l'acide acrylique au moins partiellement neutralisé, obtenue dans l'étape de procédé (ib_2), a une température située dans la plage allant de 10 à 40°C.

5. Procédé selon l'une quelconque des revendications précédentes, dans lequel le refroidisseur par absorption (3) est un refroidisseur par absorption (3) à base de sel de lithium utilisant de l'eau en tant que réfrigérant et un sel de lithium en tant qu'absorbant.

6. Procédé selon l'une quelconque des revendications précédentes, dans lequel le refroidisseur par absorption (3) comprend une coque inférieure (8) qui comprend une section d'absorbeur (9) et une section d'évaporateur (10), et une coque supérieure (4) qui comprend une section de générateur (7) et une section de condenseur (5), dans lequel la pression dans la coque inférieure (8) est située dans la plage allant de 1 à 25 mbar et la pression dans la coque supérieure (4) est située dans la plage allant de 50 à 150 mbar.

7. Procédé selon la revendication 6, dans lequel de l'eau chaude ou de la vapeur qui est générée dans au moins l'une des étapes de procédé (i) à (xi) est utilisée pour faire fonctionner le refroidisseur par absorption.

8. Procédé selon la revendication 7, dans lequel de l'eau chaude ou de la vapeur qui est générée dans l'étape de procédé (viii) est utilisée pour chauffer la solution d'absorbant diluée qui est obtenue dans la section d'absorbeur (9) après que la solution d'absorbant diluée a été transférée dans la section de générateur (7).

9. Procédé selon la revendication 7 ou 8, dans lequel de l'eau chaude ou de la vapeur qui est générée dans l'étape de procédé (xi) est utilisée pour chauffer la solution d'absorbant diluée qui est obtenue dans la section d'absorbeur (9) après que la solution d'absorbant diluée a été transférée dans la section de générateur (7).

10. Procédé selon l'une quelconque des revendications précédentes, dans lequel, dans l'étape de procédé (i), la préparation de la solution aqueuse de monomères comprend les étapes consistant à :
(ia1) neutraliser au moins partiellement de l'acide acrylique en mélangeant une première portion d'acide acrylique avec une solution aqueuse d'hydroxyde de sodium, ce qui donne ainsi une première solution aqueuse de l'acide acrylique au moins partiellement neutralisé ;
(ib1) refroidir la première solution aqueuse de l'acide acrylique au moins partiellement neutralisé avec un liquide de refroidissement dans un échangeur de chaleur (2), le liquide de refroidissement utilisé dans l'échangeur de chaleur (2) ayant été refroidi dans un refroidisseur par absorption (3) ;
(ia2) neutraliser au moins partiellement l'acide acrylique en mélangeant une deuxième portion d'acide acrylique avec la première solution aqueuse refroidie de l'acide acrylique au moins partiellement neutralisé, qui a été obtenue dans l'étape de procédé (ib1), ce qui donne ainsi une deuxième solution aqueuse de l'acide acrylique au moins partiellement neutralisé ;
(ib2) refroidir la deuxième solution aqueuse de l'acide acrylique au moins partiellement neutralisé avec un liquide de refroidissement dans un échangeur de chaleur (2), le liquide de refroidissement utilisé dans l'échangeur de chaleur (2) ayant été refroidi dans un refroidisseur par absorption (3).

11. Procédé selon la revendication 10, dans lequel, dans l'étape de procédé (ia1), entre 50 et 90 % en poids de la quantité totale d'acide acrylique dans la solution de monomères qui est polymérisée dans l'étape de procédé (iv) sont ajoutés à la solution aqueuse d'hydroxyde de sodium en tant que première portion, le reste étant ajouté en tant que deuxième portion dans l'étape de procédé (ia2).

12. Procédé selon la revendication 11, dans lequel, dans l'étape de procédé (ia1), entre 60 et 80 % en poids de la quantité totale d'acide acrylique dans la solution de monomères qui est polymérisée dans l'étape de procédé (iv) sont ajoutés à la solution aqueuse d'hydroxyde de sodium en tant que première portion, le reste étant ajouté en tant que deuxième portion dans l'étape de procédé (ia2).

13. Procédé selon l'une quelconque des revendications précédentes, dans lequel la solution aqueuse d'hydroxyde de sodium utilisée dans l'étape de procédé (ia) a une température située dans la plage allant de 25 à 55°C.

14. Procédé selon l'une quelconque des revendications précédentes, dans lequel la solution aqueuse d'hydroxyde de sodium utilisée dans l'étape de procédé (ia) a une concentration d'hydroxyde de sodium située dans la plage allant de 10 à 30 % en poids par rapport au poids total de la solution aqueuse d'hydroxyde de sodium.
